# EUROPEAN PATENT APPLICATION

(11) **EP 0 737 480 A2**
(43) Date of publication of application: **16.10.1996**
(21) Application number: 96105579.5
(22) Date of filing: 09.04.1996
(51) Int. Cl.: A61K 35/78

(54) **A method for producing an active form of incense and the associated use of the incense**

(30) Priority: 11.04.1995 IT TO950287
(71) Applicant: Fabretto, Ivano, 10097 Collegno (Torino) (IT); Pecchiai, Maria Enrica, 20124 Milano (IT)
(72) Inventor: Pecchiai, Luciano, 20124 Milano (IT); Fabretto, Luigi, 10097 Collegno (Torino) (IT)
(74) Representative: Bosotti, Luciano

(57) **Abstract**

Incense is heated so as to vaporise it with the consequent formation of the said active form, substantially without its combustion and carbonization.

## Description

### Field of the invention

The present invention generally concerns the use of incense which is a natural product obtained from Boswellia plants.

Incense consists of gum - resins and essential oils having a high terpene content (mono-terpenes, tri-terpenes and sesqui-terpenes), which, being derived from the fusion of one of more isoprene molecules, are considered to be hydrocarbons.

### Description of the prior art

The use of incense, including its use for the treatment of the human organism, dates back to antiquity and is still in widespread practice, especially in the Orient where incense is burned until it carbonizes, for example, on charcoal or in burners (sometimes, in devices usually known as censers) for ritual use, or in the form of rods or sticks.

In other cases, it is known to use the active ingredient of incense for medical purposes, that is to say pure Boswellia acid or its salified derivatives, with associated extraction techniques.

However, while burning, which continues to be the usual method of use, has the advantage of releasing incense fumes to beneficial effect, it also has the harmful consequence that it leads to the formation of carbonaceous combustion products which may be toxic. The terpenes which constitute the incense do in fact belong to the class of hydrocarbons whose combustion and carbonization releases toxic products which may even be carcinogenic. The typical blue-white colour of incense smoke is due to the combustion of these hydrocarbons.

If one bears in mind the fact that, according to recent investigations, the products of the combustion of hydrocarbons in general, and those of cigarettes in particular, are responsible for the increased incidence of asthma among more delicate individuals, particularly children who live in the homes of smokers, it is easy to understand how the practice of burning incense can have harmful effects as well as giving the desired beneficial results.

### Objects and summary of the invention

There is therefore a need to provide a solution which enables a volatile, active form of incense to be produced and which is able radically to overcome the disadvantages indicated above.

This object is achieved according to the present invention by virtue of a method having the characteristics specifically referred to in the following claims. The invention also concerns the related use.

### Detailed description of the invention

The present invention is based, essentially, on the use of the volatile fraction of incense (this term is to be understood in the present context as referring to the active ingredient and also to its derivatives, regardless of how they are obtained, for example, chemically), this volatile fraction being extracted by physical means, that is by heating, with consequent vaporisation, achieved substantially without combustion and carbonization.

In particular, the vaporisation may be achieved by heating to a temperature of the order of 60-85°C. To this end, the incense may be packaged, for example, in disposable metal containers (for example, aluminium cup-shaped capsules) containing several grams of incense intended to be heated subsequently, for example, by a timed heater (oven) similar to the ovens usually used, for example, for the diffusion of insecticides or substances such as propolis (see earlier Italian Patent No. 1 240 474).

The solution according to the invention therefore provides for the melting of the incense so as to release its active ingredients which are vaporised without, however, any form of carbonaceous combustion.

The incense-vaporisation products obtained in this way as the active volatile form are taken in via the olfactory, respiratory and percutaneous routes, as well as through the mucous membranes.

In the human and veterinary fields, the associated treatment is directed preliminarily, but not exclusively, to the treatment of inflammatory processes. As well as its use in illnesses affecting the joints, asthma, psoriasis, inflammation of the intestines and of the respiratory tract, and chronic hepatitis, it is also used more generally to strengthen the organism's defence systems as well as its psycho-physical equilibrium.

This possible generalisation of the use of the volatile fraction of incense is also due to the fact that the volatile fraction reaches the brain of the organism directly via the olfactory nerves. In addition, it is thought that it may also have a direct action against pathogenic agents such as microorganisms or parasites. It may therefore be possible to use it in agronomy as well as in the human and veterinary fields, both during cultivation(for example, of fruit, vegetables and flowers), for the prevention and treatment of processes in vegetable crops similar to inflammatory processes, and generally to strengthen the defences of vegetable crops, as well as in the preservation and storage of the final produce. Naturally, the use of an incense vaporiser according to the invention for the preservation of animal products may also be envisaged.

Finally, incense may to advantage be combined with propolis and other bee waxes in view of their similar characteristics of vaporisation without combustion and carbonization due to the common presence of terpenes.

In addition, the incorporation of flower buds and essential oils (eucalyptus, pine, cypress, juniper, sandalwood, camphor, musk etc) is envisaged, that is, those essential oils which form part of the incense paste used to make the sticks.

With regard to the typical equipment usable to obtain the active form of incense considered earlier, a 12 volt electric oven having a heating capacity of the order of 9 watts allows a tablet or pastille (capsule) of incense to be brought to a maximum temperature of 80-85°C in a period of the order of 1-1.5 minutes. The heat source of the oven is then deactivated for a period of the order of 5-10 minutes with the effect that the temperature of the residual mass of incense remains substantially within the indicated range for 5-10 minutes. At the end of this period of time, the heat source may be reactivated for a period of the order of 45-60 seconds in order to bring the propolis back to a temperature of the order of 70-75°C. The repetition of this cycle of deactivating and re-activating the heat source (possibly by means of a thermostatic system - according to known principles) enables the temperature of the incense to be maintained within the range 60-85°C in which the vaporised, active form referred to previously is obtained, the harmful phenomena of combustion/carbonisation being avoided in any case.

It is, however, understood that the aforesaid time intervals, which are linked to the power and characteristics of the heat source, although preferred, are not to be interpreted as limiting the characteristics of the present invention.

Naturally, the principle of the invention remaining the same, the details and embodiments may be varied widely with respect to that described and illustrated without thereby leaving the scope of the invention.

## Claims

1. A method for producing an active form of incense, characterised in that it comprises the step of heating the incense so as to cause its vaporisation substantially without its combustion and carbonization.

2. A method according to Claim 1, characterised in that the incense is heated to a temperature substantially between 60°C and 85°C.

3. A method according to Claim 1 or Claim 2, characterised in that it includes the step of heating the incense in cycles comprising alternate phases of heating and cooling of the incense.

4. A method according to Claim 3, characterised in that the phases of heating last for periods of the order of 45 seconds and are separated by cooling phases which last for periods of the order of 5-10 minutes.

5. The use of incense in its vaporised form, particularly, but not exclusively, for the treatment of inflammatory processes, diseases involving the joints, asthma, psoriasis, inflammation of the intestines and of the respiratory tract, and chronic hepatitis, for strengthening an organism's defence systems and its psycho-physical equilibrium against pathogenic agents such as microorganisms or parasites, in the field of agronomy, particularly during the cultivation of fruit, vegetables and flowers, for the prevention and treatment of inflammatory processes in vegetable products and generally to strengthen the defences of vegetable produce during preservation and storage, and for the preservation of animal products.

6. The use according to claim 5, with the incorporation of propolis in the incense.
